# EUROPÄISCHE PATENTSCHRIFT

(11) **EP 0 898 469 B1**
(45) Veröffentlichungstag und Bekanntmachung des Hinweises auf die Patenterteilung: **21.06.2000**
(21) Anmeldenummer: 96934652.7
(22) Anmeldetag: 11.10.1996
(51) Int. Cl.: A61F 2/30

(54) **IMPLANTAT**
IMPLANT
IMPLANT

(30) Priorität: 16.04.1996 DE 19614949
(43) Veröffentlichungstag der Anmeldung: 03.03.1999
(73) Patentinhaber: Broziat, Horst, D-2400 Lübeck (DE); Henssge, Ernst Joachim, Prof. Dr. med., 23562 Lübeck (DE)
(72) Erfinder: Broziat, Horst, D-2400 Lübeck (DE); Henssge, Ernst Joachim, Prof. Dr. med., 23562 Lübeck (DE)
(74) Vertreter: Vonnemann, Gerhard, Dr.-Ing.
(86) Internationale Anmeldenummer: EP9604439
(87) Internationale Veröffentlichungsnummer: WO9738649

(56) Entgegenhaltungen:
- EP-A- 0 162 604
- EP-A- 0 420 542
- EP-A- 0 672 395
- WO-A-95/11639
- DE-A- 4 133 877
- GB-A- 2 181 354

## Beschreibung

Die Erfindung betrifft ein Knochenimplantat mit einem Schaft zur Verankerung in einem Knochen, wobei die Oberfläche des Knochenimplantats zu gerichteten Detailformelementen ausgeformt ist. Das Verfahren zur Herstellung des Knochenimplantats ist nicht Teil der Erfindung. Die Verankerung des Implantats erfolgt durch Einwuchs des Knochens in die strukturierte Oberfläche.

Knochenimplantate, besonders solche, die Schäfte und/oder Stiele haben, sind hohen Druck-, Zug-, Biege- und Reibbeanspruchungen ausgesetzt. Die auftretenden Kräfte müssen in den Knochen des Implantatlagers abgeleitet werden. Von Knochenimplantaten wird lebenslanges Funktionieren erwartet. Knochenimplantate dürfen sich daher weder aus der Verankerung im Knochen lösen noch infolge der ständigen Wechsel-Biege-Beanspruchung brechen.

Ein derartiges Implantat mit strukturierter Schaftoberfläche ist in der EP 0 128 036 beschrieben. Das Implantat weist im breiteren oberen Teil des Schaftes eine zirkuläre strukturierte Oberfläche auf, während die Oberfläche der unteren Anteile glatt, d. h. nicht strukturiert ist. Durch die Unregelmäßigkeit der das Niveau des Schaftes überragenden strukturierten Oberfläche können Probleme beim Eintreiben des Schaftes in die Markhöhle des Knochens, z. B. des Oberschenkelknochens auftreten, was zu unerwünschten Spannungsspitzen mit Knochenschädigung bis hin zur Möglichkeit der Sprengung des Knochenrohres führen kann.

Bei der Herstellung eines Implantats wird üblicherweise zunächst eine verlorene Positivform des Implantats erstellt. Die strukturierte Oberfläche wird erzeugt, indem an den entsprechenden Abschnitten beispielsweise offenporige Schwämme, retikulierter Filterschaum oder tetrapodenartige Körper in einem zweiten Arbeitsgang auf der Oberfläche der Positivform befestigt werden. Dies können natürliche Schwämme, retikulierter Filterschaum oder tetrapodenartige Körper sein, die durch Eintauchen in eine Wachslösung oder -emulsion und anschließendes Aushärten für das Ausschmelzen im Feingußverfahren präpariert werden. Von der fertigen Positivform wird dann eine Negativform hergestellt, die meist aus Keramik besteht und durch Brennen ausgehärtet wird. Beim Brennen schrumpft die Form. Entsprechender Schwund muß bei der Herstellung der Positivform berücksichtigt werden. Nach Ausschmelzen der verlorenen Positivform wird die Negativform mit flüssigem, körperverträglichem Metall oder einer Legierung gefüllt. Ist das Metall abgekühlt und erstarrt, wird die Negativform abgeschlagen und das Implantat feinbearbeitet. Reste der Negativform werden chemisch oder durch Sandstrahlen aus der strukturierten Oberfläche entfernt, die Bereiche mit nicht strukturierter Oberfläche können poliert werden.

So wird in der DE C 41 33 877 ein Verfahren offenbart, bei dem ein Grundkörper aus Modellmaterial geformt wird, der mindestens abschnittsweise mit einem porenhaltigen Gewirke, Gewebe oder Geflechte aus Modellmaterial belegt wird, welches eine vorgegebene Porenstruktur aufweist.

Diese Herstellverfahren sind jedoch aufwendig, wodurch die Implantate sehr teuer werden. Es muß für jedes Implantat eine individuelle verlorene Form aus Wachs oder Kunststoff erstellt werden. Die Bereiche strukturierter Oberfläche unterliegen in ihrem Aufbau einer Toleranz, die durch die natürlichen Schwankungen des offenporigen wachsbeschichteten Materials verursacht werden. Gewünscht wird jedoch ein standardisierter Aufbau der Oberflächenstruktur, um eine reproduzierbare Qualität des Implantats zu erreichen.

Es ist daher Aufgabe der Erfindung, ein Knochenimplantat zur Verfügung zu stellen, bei dem die Kontaktzone der Werkstoffe besonders gute mechanische Übertragungseigenschaften aufweist und kostengünstig zu fertigen ist. Form und Art der Oberflächenstruktur soll sich ohne großen Aufwand anpassen lassen. Insbesondere sollen entsprechend geformte Implantate herstellbar sein, die sich kurzfristig und fest durch einsprossendes Knochengewebe verankern lassen.

Die Vorrichtungsaufgabe wird durch die Merkmale gemäß Anspruch 1 gelöst.

Als besonders dauerhaft hat sich der Übergangsbereich zwischen Knochen und Implantat erwiesen, wenn der Abstand der Detailformelemente das 1- bis 5-fache und/oder der Überstand das 1- bis 15-fache eines typischen Querschnittsmaßes, vorzugsweise des kleinsten Querschnittsmaßes, der Detailformelemente beträgt. Durch diese Dimensionierung werden nachteilige Spannnungskonzentrationen vermieden.

Für Implantate ist eine Dimensionierung vorteilhaft, bei der die Detailformelemente ein typisches kleinstes Querschnittsmaß von 0,3 bis 1,5 mm und/oder typisches Abstandsmaß von 1,5 bis 4,5 mm aufweisen und/oder einen typischen Überstand von 1,5 bis 4,5 mm aufweisen.

Sind die Datailformelemente auf mindestens einer Teilfläche im Bereich höchster Zug- oder Druckbelastung auf der Oberfläche des Schaftes angeordnet, kann das Implantat beim Einschlagen entlang der unstrukturierten Teilfläche im vorpräparierten Kanal in den Knochen eingleiten. Spannungsspitzen im Knochen werden weiter vermindert, oder gar vermieden, wenn mindestens eine Teilfläche mit strukturierter Oberfläche gegenüber der unstrukturierten Oberfläche zurückgesetzt ist. Die Implantate können auch mit einer zurückgesetzten Fläche gefertigt werden, die von den Detailformelementen netzartig überspannt werden. Auf diese Weise kann der Knochen in die hinter dem Netz gebildete Hohlkammer einsprießen. Für das Implantat ergibt sich dadurch eine gemeinsame stetige Hüllfläche, die vom Vollkörper gleichmäßig in die strukturierten Bereiche übergeht, z.B. mit netz- oder brückenartiger Struktur, so daß sich durch diese spezielle Formgebung der Struktur eine Statik ergibt, die etwa der des Vollkörpers gleichkommt.

Als weiterer Vorteil ergibt sich, daß das Implantat, im Gegensatz zu Implantaten mit umlaufender strukturierter Oberfläche, beispielsweise mittels fluoreszierender Benetzungsmittel, Röntgenstrahlen oder Ultraschall zerstörungsfrei auf Materialfehler untersucht werden kann. Die Gefahr des vorzeitigen Bruchs des implantierten Implantats wegen Materialermüdung kann somit verringert werden. Die Materialprüfung kann auch standardisiert werden, was für eine Zertifizierung des Herstellbetriebs nach DIN/ISO 9000 gefordert wird.

Außerdem kann die Oberfläche des Implantats problemlos nachbearbeitet werden. Unebenheiten oder Werkzeugspuren lassen sich entfernen und die Oberfläche kann poliert werden.

Eine ausreichende Fixierung des Implantats im Knochen wird erreicht, wenn die Detailformelemente auf mindestens zwei Teilflächen der Schaftoberfläche angeordnet sind, vorzugsweise an einander gegenüberliegenden Seiten des Schaftes. Dies bedeutet beispielsweise bei anatomisch angepaßten Implantaten, die entsprechend gekrümmt sind, daß die Teilflächen mit der durch die Detailformelemente gebildete strukturierten Oberfläche auf der konvex gekrümmten Seite bzw. auf der konkav gekrümmten Seite des Schaftes angeordnet sind. Durch diese Anordnung wird eine gute Ableitung der Kräfte aus dem Implantat in den umliegenden Knochen erzielt. Außerdem erlauben die gegenüberliegenden unstrukturierten Teilflächen ein störungsfreies Durchstrahlen des Implantats mit Röntgenstrahlen zur Materialprüfung.

Eine innige Verbindung von Knochen und Implantat wird erzielt, wenn die mindestens eine Teilfläche mit strukturierter Oberfläche zwischen 15 - 60% der Schaftlänge bedeckt.

Die Breite der strukturierten Teilfläche wird dabei günstig so gewählt, daß die mindestens eine Teilfläche mit strukturierter Oberfläche am Ort ihrer größten Breite in Umfangsrichtung eine Ausdehnung zwischen 10 - 45% des Umfangs aufweist. Durch die größere Angriffsfläche und den größeren Abstand zur Schaftachse werden die zu übertragenden Kräfte geringer und können auf eine größere Fläche verteilt werden.

Sind die Verbindungsstege der Detailformelemente entlang der Längs- und / oder der Umfangsrichtung ausgerichtet, wird das Eintreiben des Implantats weiter erleichtert. Das Implantat gleitet sowohl auf den unstrukturierten Teilbereichen, wie auch entlang der längs ausgerichteten Detailformelemente.

Spannungsspitzen im Knochen während des Eintreibens werden weiter verringert oder gar vermieden, wenn die mindestens eine Teilfläche mit strukturierter Oberfläche gegenüber der übrigen unstrukturierten Oberfläche zurückgesetzt ist.

Die Rücksetzung mindestens einer Teilfläche mit strukturierte Oberfläche wird vorteilhafterweise so bemessen, daß die mindestens eine Teilfläche in Längsrichtung eine gemeinsame stetige Hüllfläche mit dem Implantatschaft aufweisen. Das Implantat gleitet dann sowohl auf den unstrukturierten Teilflächen wie auch auf den äußeren Abschnitten der Detailformelemente. Die Spannungen können mit geringer Umlenkung aus dem vollen Material in die verbindenden Stege geleitet werden, so daß nur geringe Kerbspannungen an den Übergängen zu erwarten sind. Diese Maßnahme erhöht somit die Standzeit des Implantats.

Bei einem geraden oder anatomisch adaptierten Implantat mit gekrümmtem Schaft wird die strukturierte Teilfläche vorteilhaft so gewählt, daß auf der Seite konvexer Krümmung die Teilfläche eine Länge von 25-60% der Schaftlänge aufweist. Die konvexe Seite, die auch als "Rücken" des Implantats bezeichnet, ist meist die zugbelastete Seite. Die gegenüberliegende konkave Seite wird entsprechend mit "Bauch" bezeichnet.

Desweiteren kann die Detailformelemente aufweisende konkave Teilfläche auch vorteilhaft so gestaltet sein, das die Teilfläche eine Länge von 15-40% der Schaftlänge aufweist.

Da nur in die strukturierten Teilflächen Knochen einwächst, werden für die Kraftübertragung zwischen Implantat und Knochen nur die Bereiche genutzt, in denen die höchsten Beanspruchungen auftreten. Die neutrale Belastungszone des Implantats wird von Oberflächenstrukturen freigehalten.

Die Einsprossung von Knochenmaterial wird erleichtert, wenn das Knochenimplantat aus Titan, oder einer Legierung davon, vorzugsweise einstückig, gegossen ist. Überraschenderweise läßt die Form der Detailformelemente auch einen Guß in Titan oder einer Titanlegierung zu, was bei anderen Geometrien nicht möglich ist. Die bekannten Spongiosa-Metall-Implantate können im Feinguß nur aus Legierungen auf Kobaltbasis oder Edelstahllegierungen, nicht aber aus Titanlegierungen, hergestellt werden.

Weitere vorteilhafte Ausgestaltungen sind in den Unteransprüchen 13 und 14 erläutert.

Bei herkömmlichen Verfahren zur Herstellung eines Knochenimplantats wird von einem Positivmodell, das dem Knochenimplantat entspricht, eine Herstellform, die einem Negativmodell des Knochenimplantats entspricht, angefertigt. Danach wird die Herstellform mit einem körperverträglichen Material ausgefüllt und nach dem Verfestigen des Materials die Form entfernt. Die Verfahrensaufgabe wird dadurch gelöst, daß zunächst ein vom Hauptkörper des Positivmodells des Implantats getrenntes positives Oberflächenstrukturmodell und/oder von der Herstellform getrennte negative Teilform für den Bereich der strukturierten Oberfläche hergestellt wird. Das Oberflächenstrukturmodell wird anschließend in vorgesehene Aussparungen des Positivmodells bzw. in die Teilform in entsprechende Aussparungen der Herstellform montiert wird, bevor das Material in die Form gegossen.

Sowohl Oberflächenstrukturmodell als auch negative Teilform können standardisiert werden und beispielsweise von einer Urform in größeren Serien abgeformt werden. Außerdem können negative Teilformen bzw. Oberflächenstrukturmodelle mit unterschiedlichen Oberflächenstrukturen erstellt werden. Auf diese Weise können durch Zusammenfügen der negativen Teilform und der Herstellform bzw. des Oberflächenstrukturmodells und des positiven Hauptkörpers einfach und kostengünstig Knochenimplantate erstellt werden, deren Oberflächenstruktur den jeweiligen Anforderungen der Anwender entspricht.

Beispielsweise läßt sich ein plattenförmiges Keramikteil im grünen Zustand durch Stanzen oder Wasserstrahlverfahren mit Löchern versehen. Zwischen den Löchern werden verbindende Furchungen eingeformt. Legt man ein solches, getrenntes Teil so in eine negative Herstellform, daß die Furchungen zur Form weisen und die Löcher der Öffnungen an der Oberfläche liegen, dann bildet sich nach dem Guß eine netzartige Struktur aus. Zu diesem Zweck wird das Gießmaterial entgegen der Stanzrichtung in die Öffnungen der Stanzungen geleitet. Es lassen sich auf diese Weise auch einzeln stehende, beispielsweise pilzförmige, Strukturen mit Hintergriffen erzeugen.

Hintergriffartige, brücken- oder netzartige Strukturen können geschaffen werden, indem in die Teilform durchdringende Kanäle eingestanzt werden. Beim Ausgießen der Form entstehen dann durch das Ausfüllen der Kanäle Stege, die durch Elemente der Oberflächenstruktur so verbunden werden, daß beispielsweise brückenartige Detailformelemente entstehen.

Die Herstellung der Knochenimplantate läßt sich standardisieren, was für eine Zertifizierung nach DIN/ISO 9000 gefordert wird. Das Verfahren kann so durchgeführt werden, daß die Teilform zum Erzeugen der Oberflächenstruktur für das verlorene Positivmodell verwendet wird. Die Oberflächenstruktur wird dann am Positivmodell montiert. Zur Gewährleistung der erforderlichen Wechsel-Biege-Festigkeit des Implantatschaftes ist die Tiefe der Teilstrukturbereiche so zu gestalten, daß der zurückgesetzte strukturierte Teil zum Schaftende hin fließend in den nicht strukturierten Teil des Schaftes übergeht.

Durch geeignete Ausgestaltung der Teilform kann das Verfahren jedoch auch so durchgeführt werden, daß die Teilform in das Negativmodell, also die Herstellform, montiert wird.

Bei Verwendung einer Positivform wird dabei vorteilhafterweise so verfahren, daß die Positivform aus verflüssig- oder verflüchtigbarem Material hergestellt wird. Besonders geeignet sind Wachs und Kunststoff.

Die Erfindung wird in einer bevorzugten Ausführungsform unter Bezugnahme auf eine Zeichnung beschrieben, wobei weitere vorteilhafte Einzelheiten den Figuren der Zeichnung zu entnehmen sind. Funktionsmäßig gleiche Teile sind dabei mit denselben Bezugszeichen versehen.

Die Figuren zeigen im einzelnen:
- Fig.1: Eine Seitenansicht des Knochenimplantats
- Fig.2: Einen Schnitt durch den Schaft des Implantats entlang der Linie II - II in Fig. 1.
- Fig.3: Eine Detailanschicht der Oberflächenstruktur.
- Fig.4: Einen Schnitt durch eine Negativform der zweiten Teilform.
- Fig.5: Eine schematisierte Darstellung einer Negativform der ersten Teilform.
- Fig.6: Eine schematisierte Darstellung der zweiten Teilform als Positivform.
- Fig.7: Eine Darstellung der ersten Teilform als Positivform.
- Fig.8: Einen Schnitte durch ein Geradschaftimplantat.
- Fig.9: Einen Schnitt durch ein anatomisch geformtes Implantat.

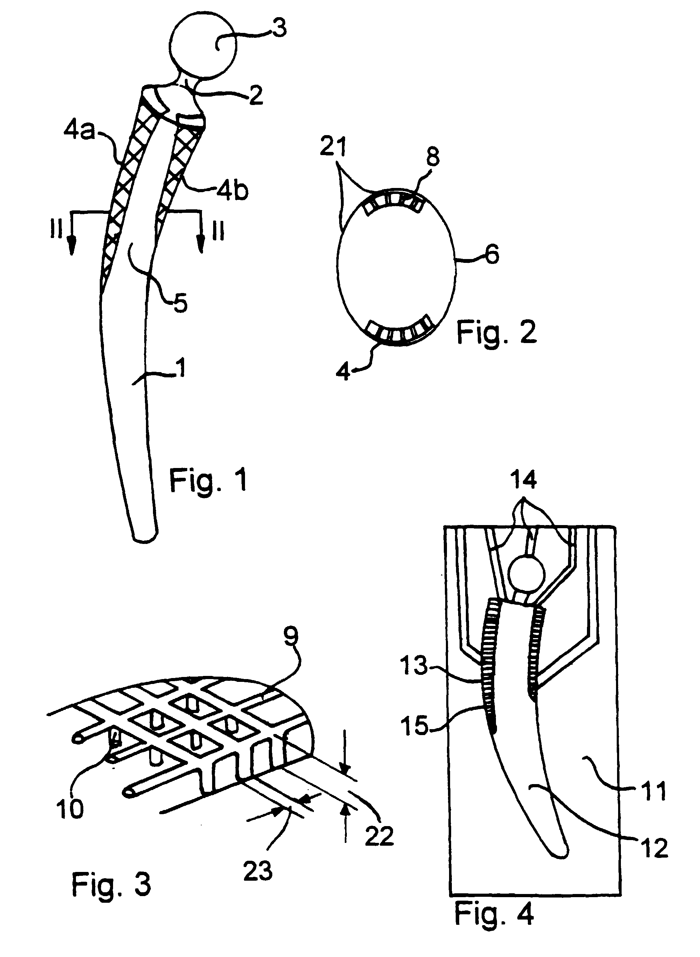

Figur 1 zeigt ein Knochenimplantat zum Ersatz eines Hüftgelenkes. Das Implantat weist einen keilförmigen Schaft 1 auf, der sich an seinem Ende mit dem größeren Durchmessers zu einem Hals 2 verjüngt, an den sich die Gelenkkugel 3 anschließt. Das Implantat ist anatomisch adaptiert. Der Schaft weist deshalb eine Krümmung auf. Der Schaft weist im wesentlichen eine glatte Oberfläche auf. Diese ist im allgemeinen poliert, kann aber auch durch Bearbeitung leicht aufgerauht sein. In der Schafthälfte mit größerem Durchmesser sind zwei Bereiche mit strukturierter Oberfläche 4 angeordnet.

Der erste Teilbereich 4a ist auf der Seite konvexer Krümmung angeordnet. Die Teilfläche 4a entspricht dem Bereich höchster Zugbelastung des Schaftes. In diesem Bereich wird der überwiegende Teil der Kräfte aus dem Knochenimplantat in den Knochen eingeleitet. Die Längsausdehnung der Teilfläche 4a entspricht ungefähr 35 bis 40 % der Schaftlänge. Sie ist zu ihrer Längsachse symmetrisch, und zeigt an ihrer breitesten Stelle eine Ausdehnung von za. 20 bis 30 % des Schaftumfangs. Auf der gegenüberliegenden, konkaven Seite des Schaftes ist eine zweite Teilfläche 4b angeordnet. Die zweite Teilfläche 4b entspricht dem Bereich höchster Druckbelastung des Schaftes. Auch in diesem Bereich kann durch die Verwachsung mit dem Knochen die größte Kraft aus dem Implantat abgeleitet werden. Ihre Längsausdehnung entspricht ungefähr 20 % der Schaftlänge. Der Schwerpunkt der Fläche befindet sich ungefähr in der Mitte des oberen Drittels des Schaftes. An ihrer breitesten Stelle hat die zweite Teilfläche 4b eine Ausdehnung von ungefähr 20 % des Schaftumfangs. Im neutralen Bereich 5, in dem geringere Druck- und Zugspannungen auftreten, ist die Schaftoberfläche unstrukturiert. Strukturierte Bereiche und unstrukturierte Bereiche weisen eine gemeinsame Hüllfläche auf. Auf diese Weise werden Spannungsspitzen beim Eintreiben des Implantats in den Knochen vermieden.

Fig. 2 zeigt einen Schnitt durch den Schaft des Implantats entlang der Markierung II-II. Der Schaft zeigt einen annähernd ovalen Querschnitt, wobei sich in Umfangsrichtung Bereiche strukturierter Oberfläche 4 und Bereiche mit glatter Oberfläche 6 abwechseln. Die strukturierte Fläche 4 ist gegenüber der glatten Oberfläche 6 zurückgesetzt, so daß die Detailformelemente 8 und die glatte Oberfläche 6 eine gemeinsame Hüllfläche 21 ergeben. In der dargestellten Ausführungsform ist der Schaft massiv ausgeführt, es ist jedoch auch möglich, innerhalb des Schaftes Hohlräume vorzusehen. Ebenso können in den Bereichen mit glatter Oberfläche Längsrippen vorhanden sein, die der Führung des Implantats beim Eintreiben dienen.

Fig. 3 zeigt eine Detailansicht einer speziellen Ausführungsform der Detailformelemente. Aus entlang der Umfangsrichtung und der Längsrichtung verlaufenden Stegen 9 wird eine netzartige Struktur gebildet, diese Struktur wird von senkrechten Stegen 10 auf dem Schaft abgestützt. Der Überstand 22 beträgt ungefähr das fünffache des Querschnittsmaßes 23 der Stege 10. In die Zwischenräume zwischen den Stegen kann Knochenmaterial einwachsen, wobei der Knochen die Stege 9 hintergreift, und so eine innige Verbindung zwischen Implantat und umgebenden Knochen geschaffen werden.

Fig. 4 zeigt einen Schnitt durch eine Gießform. Diese kann aus Keramik, Sand oder ähnlichem hergestellt sein. Die Gießform ist mehrteilig aufgebaut und besteht aus einer Herstellform 11 und darin eingesetzten Teilformen 15. Die Herstellform 11 enthält eine Negativform 12 des Knochenimplantats. In der Herstellform 11 sind Aussparungen 13 vorgesehen, die den Bereichen mit strukturierter Oberfläche entsprechen. In diese Aussparungen 13 werden strukturierte Teilformen 15 eingesetzt. Die Gießform weist weiter Kanäle 14 auf, durch die das flüssige Metall eingefüllt werden bzw. Gas entweichen kann.

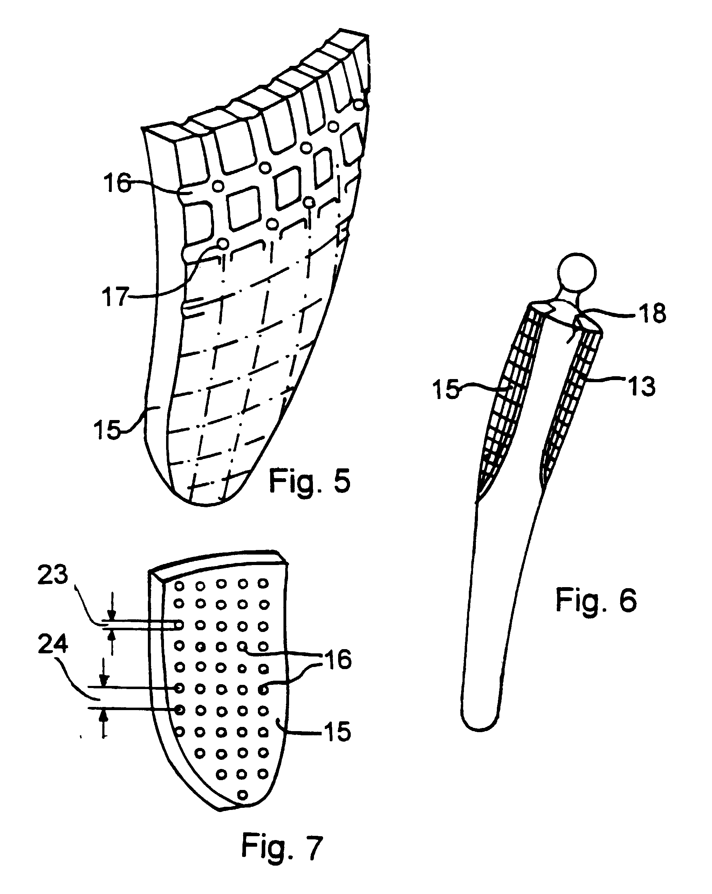

Fig. 5 zeigt eine Teilform 15, die vorzugsweise aus Keramik hergestellt wird. Vor dem Gießen des Implantats wird das Teil in die zugehörige Aussparung 13 der in Fig. 4 gezeigten Herstellform 11 eingesetzt. Auf einer Seite der Teilform 15 ist eine Struktur 16 eingeprägt, die dem Negativ der strukturierten Oberfläche 4 des Implantats entspricht. In der gezeigten Ausführungsform ist dies ein netzförmiges Muster. Die gezeigte Ausführungsform weist Kanäle 17 auf, die den in Fig. 3 gezeigten Stegen 10 zur Verbindung der netzförmigen Struktur mit dem Schaftkörper entsprechen. Die Herstellung der Form erfolgt in der Weise, daß zunächst ein Rohling aus einer grünen Masse knetbarer Konsistenz geformt wird, auf dessen einer Seite dann eine perforierende Struktur 16, 17 eingeprägt wird. Der Rohling wird dann durch Trocknen und Brennen ausgehärtet. Der Zusammenbau der Gießform erfolgt durch Einlegen der Teilstücke 15 in die Aussparungen 13 der Herstellform 11. Dabei kommt die strukturierte Seite des Teilstücks 15 an der Herstellform 11 zur Anlage. Nach dem Ausgießen der Form und Erkalten des Metalls wird die Gießform abgeschlagen und noch vorhandene Reste der Gießform in den strukturierten Bereichen durch Sandstrahlen oder andere Maßnahmen entfernt.

Fig. 6 zeigt ein aus mehreren Teilstücken zusammengesetztes Positivmodell eines Knochenimplantats. Dieses Positivmodell wird aus einem leicht schmelzbaren oder flüchtigem Material, z.B. Wachs hergestellt. Das Modell besteht aus einem Hauptkörper 18, der Aussparungen 13 aufweist, in die Teilstücke 15 mit strukturierter Oberfläche eingelegt werden können.

Das Teilstück 15 ist in Fig. 7 genauer gezeigt. Es weist auf einer Seite eine Struktur 16 auf. In der gezeigten Ausführungsform sind dies kreisrunde noppenförmige Vertiefungen. Der Abstand 24 der Vertiefungen 16 beträgt ungefähr das fünffache ihres Querschnitts 23.

Zum Zusammenbau werden die Teilstücke 15 in die Aussparungen 13 des Hauptkörpers 18 eingelegt. Von diesem wird dann eine Negativform beispielsweise aus Keramik abgenommen. Nach Ausschmelzen der Positivform wird dann eine entsprechende Gießform zur Herstellung von Knochenimplantaten erhalten. Gießvorgang und Nachbearbeitung werden dann wie oben beschrieben durchgeführt.

Auf diese Weise ist ein Knochenimplantat geschaffen worden, das mit geringen Schwierigkeiten in den vorbereiteten Knochen eingetrieben werden kann und das durch Verwachsen mit dem Knochen zuverlässig im Schaft fixiert werden kann. Ferner ist ein Verfahren zur Herstellung eines Knochenimplantats geschaffen worden, mit dem Knochenimplantate kostengünstig und unter standardisierten Bedingungen in größeren Serien erstellt werden können und bei dem durch Kombination von standardisierten Teilformen das Implantat einfach an die gewünschten Eigenschaften angepaßt werden können.

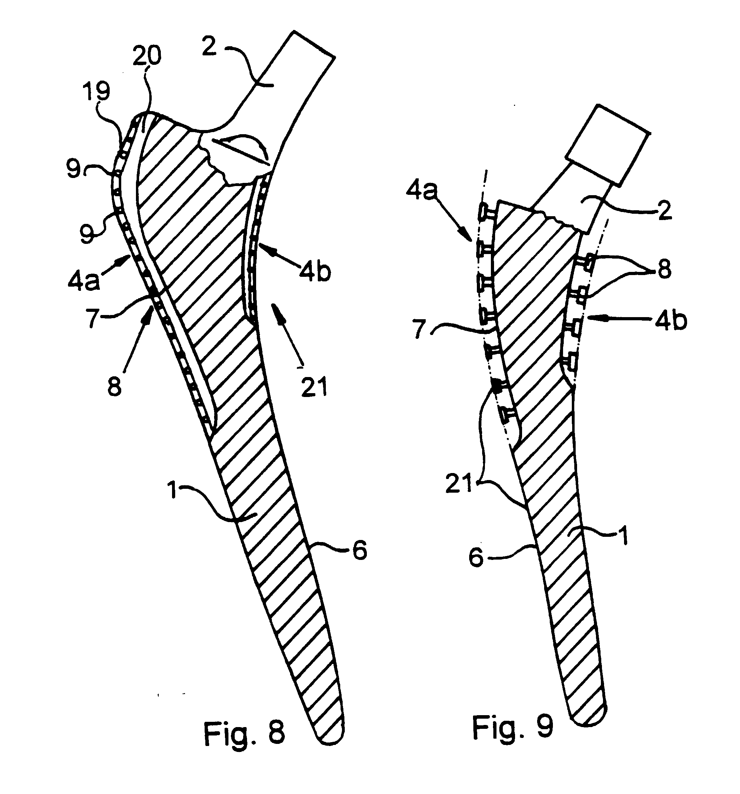

Solche fertigen Implantate sind, teilweise als Schnitt, in den Figuren 8 und 9 dargestellt.

Das Geradschaftimplantat gemäß Figur 8 weist am oberen, dem Hals 2 zugewandten Ende des Schaftes 1 eine zurückgesetzte Fläche 7 auf. Diese Fläche 7 wird von einer Netzstruktur 19 überspannt. Dadurch ergibt sich zwischen Netzstruktur 19 und zurückgesetzter Fläche 7 ein Hohlraum 20, in den der Knochen einwachsen kann. Die Netzstruktur geht seitlich in den Schaft 1 über. In diesem Fall sind also keine zusätzlichen Verbindungsstege vom Netz zur Fläche 7 vorgesehen. In der Darstellung kann man somit die Stege 9 der Netzstruktur sowohl im Schnitt wie auch von der Seite erkennen. Die Netzstruktur 19 und der Schaft 1 weisen eine gemeinsame Hüllfläche 21 auf.

Das in Figur 9 gezeigte anatomisch geformte Implantat weist im Bereich mit strukturierter Oberfläche 4 Detailformelemente 8 auf, die pilzförmig ausgebildet sind. Die strukturierte Fläche 7 ist gegenüber der glatten Oberfläche 6 des Schaftes 1 zurückgesetzt, so daß sich dann wiederum eine gemeinsame Hüllfläche 21 für die aus Fläche 7 vorstehenden Detailformelemente 8 und der glatten Oberfläche 6 des Schaftes 1 ergibt.

Die pilzförmigen Elemente können jedoch auch nur teilweise versenkt sein.

### Bezugszeichenliste

- 1: Schaft
- 2: Hals
- 3: Gelenkkugel
- 4a,b: Teilbereich mit strukturierter Oberfläche
- 5: neutraler Bereich
- 6: Bereich mit glatter Oberfläche
- 7: strukturierte Fläche
- 8: Detailformelemente
- 9: Steg
- 10: Steg
- 11: Herstellform
- 12: Negativform
- 13: Aussparungen
- 14: Kanäle
- 15: Teilform
- 16: Struktur, Furchungen
- 17: Kanäle, Öffnungen
- 18: Hauptkörper
- 19: Netzstruktur
- 20: Hohlraum
- 21: Hüllfläche
- 22: Überstand
- 23: Querschnittsmaß
- 24: Abstand
- 100: Abschnitt
- 102: Hüftpfanne
- 103: Pol
- 104: Formteil
- 105: Oberfläche, Außenfläche
- 106: Einsatz
- 107: Rand
- 109: Kurve
- 112, 112': Detailformelement, Ansatz
- 114, 114': Stiel
- 116, 116': Kopf
- 118: Außenfläche
- 122: Haftansatz
- 124: Stiel
- 126: Kopf
- 130: Schneidanker
- 140: Zwischenräume
- α: Winkel
- A: Abstand
- L, 1: Länge
- L, 1, α, A und D: Parameter

## Patentansprüche

1. Knochenimplantat mit einem Schaft (1) zur Verankerung in einem Knochen, wobei die Oberfläche des Knochenimplantats zu gerichteten Detailformelementen (8) ausgeformt ist, die zum freien Ende hin verdickend geformt sind und/oder teilweise brückenartig und/oder netzartig durch Verbindungsstege (10) verbunden sind, , **dadurch gekennzeichnet**, daß die Detailformelemente (8) auf nur zwei Teilflächen (4a,b) angeordnet sind, wobei die erste auf der lateralen Seite konvexer Krümmung und die zweite auf der gegenüberliegenden, medialen, konkaven Seite des Schaftes liegt.

2. Knochenimplantat nach Anspruch 1, **dadurch gekennzeichnet,** daß ein Abstand (24) der Detailformelemente (8) das 1- bis 5-fache und/oder ein Überstand (22) das 1- bis 15-fache eines typischen Querschnittsmaßes (23), vorzugsweise des kleinsten Querschnittsmaßes (23), der Detailformelemente (8) beträgt.

3. Knochenimplantat nach Anspruch 1 oder 2, **dadurch gekennzeichnet**, daß die Detailformelemente (8) ein typisches kleinstes Querschnittsmaß (23) von 0,3 bis 1,5 mm und/oder typisches Abstandsmaß (24) von 1,5 bis 4,5 mm aufweisen und/oder einen typischen Überstand (22) von 1,5 mm bis 4,5 mm aufweisen.

4. Knochenimplantat nach Anspruch 1, 2 oder 3, **dadurch gekennzeichnet**, daß die Teilflächen (4a,b) zwischen 15 - 60% der Schaftlänge bedecken.

5. Knochenimplantat nach Anspruch 1, 2, 3 oder 4, **dadurch gekennzeichnet**, daß die zwei Teilflächen (4a,b) am Ort ihrer größten Breite in Umfangsrichtung eine Ausdehnung zwischen 10 - 45% des Umfangs aufweisen.

6. Knochenimplantat nach Anspruch 1, 2, 3, 4 oder 5, **dadurch gekennzeichnet**, daß die Verbindungsstege (9) der Detailformelemente (8) entlang der Längs- und / oder der Umfangsrichtung ausgerichtet sind.

7. Knochenimplantat nach Anspruch 1, 2, 3, 4, 5 oder 6, **dadurch gekennzeichnet**, daß mindestens eine der Teilflächen (4a,b) gegenüber der übrigen Oberfläche (6) zurückgesetzt ist.

8. Knochenimplantat nach Anspruch 1, 2, 3, 4, 5, 6 oder 7, **dadurch gekennzeichnet**, daß mindestens eine der Teilflächen (4a,b) und die übrige Fläche (6) in Längsrichtung des Schaftes eine gemeinsame stetige Hüllfläche (21) aufweisen.

9. Knochenimplantat nach Anspruch 1, 2, 3, 4, 5, 6, 7 oder 8, **dadurch gekennzeichnet**, daß die gegenüber der übrigen Fläche (6) zurückgesetzte Teilfläche (7), unter Ausbildung einer Hohlkammer (20) von den Detailformelementen (19) netzartig überspannt wird.

10. Knochenimplantat nach einem oder mehreren der vorhergehenden Ansprüche, **dadurch gekennzeichnet**, daß die auf der Seite konvexer Krümmung angeordnete Teilfläche (4a) eine Länge von 25-60% der Schaftlänge aufweist.

11. Knochenimplantat nach einem oder mehreren der vorhergehenden Ansprüche, **dadurch gekennzeichnet**, daß die auf der Seite konkaver Krümmung angeordnete Teilfläche (4b) eine Länge von 15-40% der Schaftlänge aufweist.

12. Knochenimplantat nach einem oder mehreren der vorhergehenden Ansprüche, **dadurch gekennzeichnet**, daß das Knochenimplantat aus einem bioinerten Material, insbesondere einer Titanlegierung, vorzugsweise einstückig, gegossen ist.

13. Knochenimplantat nach einem oder mehreren der vorhergehenden Ansprüche, **dadurch gekennzeichnet**, daß die strukturierten Teilflächen und die übrigen Flächen so angeordnet sind, daß das Implantat einer zerstörungsfreien Materialprüfung unterziehbar ist.

14. Knochenimplantat nach einem oder mehreren der vorhergehenden Ansprüche, **dadurch gekennzeichnet**, daß die Detailformelemente (8) verschieden geformte Stiele (14) und/oder verschieden geformte Köpfe (16) aufweisen.

## Claims

1. A bone implant with a shaft (1) for anchoring in a bone, whereby the surface of the bone implant is shaped to form specific detailed mould elements (8), which are shaped so as to thicken towards the free end and/or are connected in part in bridge fashion and/or in mesh fashion by connecting webs (10), characterised in that the detailed mould elements (8) are arranged on only two part surfaces (4a, b), whereby the first is located on the lateral side of the shaft with convex curvature, and the second is located on the opposite medial concave side of the shaft.

2. A bone implant according to claim 1, characterised in that an interval (24) between the detailed mould elements (8) amounts to 1 to 5 times a typical cross-section (23), and/or a projection (22) amounts to 1 to 15 times a typical cross-section (23), for preference the smallest cross-section dimension of the detailed mould elements (8).

3. A bone implant according to claim 1 or 2, characterised in that the detailed mould elements (8) feature a typical smallest cross-section dimension (23) of 0.3 to 1.5 mm and/or typical interval dimension (24) of 1.5 to 4.5 mm and/or a typical projection (22) of 1.5 to 4.5 mm.

4. A bone implant according to claim 1, 2 or 3, characterised in that the part surfaces (4a, b) covers between 15-60 % of the shaft length.

5. A bone implant according to claim 1, 2, 3 or 4, characterised in that the two part surfaces (4a, b), at the point of their broadest width in the circumferential direction, feature an expansion of between 10 and 45 % of the circumference.

6. A bone implant according to claim 1, 2, 3, 4 or 5, characterised in that the connection webs (9) of the detailed mould elements (8) are directed along the longitudinal and/or circumferential direction.

7. A bone implant according to claim 1, 2, 3, 4, 5 or 6, characterised in that at least one of the part surfaces (4a, b) are recessed in relation to the remaining surface (6).

8. A bone implant according to claim 1, 2, 3, 4, 5, 6 or 7, characterised in that at least one of the part surfaces (4a, b) and the remaining surface (6) feature a common envelope surface (21) in the longitudinal direction of the shaft.

9. A bone implant according to claim 1, 2, 3, 4, 5, 6, 7 or 8, characterised in that the part surface (7), recessed in relation to the remainder of the surface (6), is spanned in mesh fashion by the detailed mould elements (19), forming a hollow chamber (20).

10. A bone implant according to one or more of the foregoing claims, characterised in that the part surface (4a) arranged on the side with the convex curvature features a length of 25-60 % of the shaft length.

11. A bone implant according to one or more of the foregoing claims, characterised in that the part surface (4b) arranged on the side with the concave curvature features a length of 15-40 % of the shaft length.

12. A bone implant according to one or more of the foregoing claims, characterised in that the bone implant is cast from a bio-inert material, in particular a titanium alloy, and for preference as a single piece.

13. A bone implant according to one or more of the foregoing claims, characterised in that the structured part surfaces and the remaining surfaces are arranged in such a way that the implant can be subjected to nondestructive material testing.

14. A bone implant according to one or more of the foregoing claims, characterised in that the detailed mould elements (8) feature shafts (14) of differing forms and/or heads (16) of differing forms.

## Revendications

1. Implant d'ostéosynthèse comprenant une tige (1) pour ancrage dans un os, la surface de l'implant d'ostéosynthèse étant façonnée de manière à présenter des éléments de détail moulés (8) orientés s'élargissant vers leur extrémité libre et/ou étant reliés partiellement, en formant des passerelles et/ou un filet, par des barrettes de liaison (10), caractérisé en ce que les éléments de détail moulés (8) ne sont agencés que sur deux faces partielles (4a, b) dont la première est située du côté latéral d'incurvation convexe, et la seconde, du côté concave médian opposé de la tige.

2. Implant d'ostéosynthèse selon la revendication 1, caractérisé en ce qu'un écartement (24) des éléments de détail moulés (8) est de 1 à 5 fois et/ou une extrémité en porte à faux (22) est de 1 à 15 fois une dimension typique de section transversale (23), de préférence de la plus petite dimension de section transversale (23) des éléments de détail moulés (8).

3. Implant d'ostéosynthèse selon la revendication 1 ou 2, caractérisé en ce que les éléments de détail moulés (8) présentent une plus petite dimension de section transversale typique (23) de 0,3 à 1,5 mm et/ou une dimension d'écartement typique (24) de 1,5 à 4,5 mm et/ou un porte à faux (22) typique de 1,5 mm à 4,5 mm.

4. Implant d'ostéosynthèse selon la revendication 1, 2 ou 3, caractérisé en ce que les faces partielles (4a, b) occupent 15 à 60 % de la longueur de la tige.

5. Implant d'ostéosynthèse selon la revendication 1, 2, 3 ou 4, caractérisé en ce que les deux faces partielles (4a, b) présentent à l'endroit de leur plus grande largeur dans la direction périphérique, une étendue comprise entre 10 et 45 % du pourtour.

6. Implant d'ostéosynthèse selon la revendication 1, 2, 3, 4 ou 5, caractérisé en ce que les barrettes de liaison (9) des éléments de détail moulés (8) sont orientés suivant la direction longitudinale et/ou la direction périphérique.

7. Implant d'ostéosynthèse selon la revendication 1, 2, 3, 4, 5 ou 6, caractérisé en ce qu'au moins l'une des faces partielles (4a, b) est en retrait par rapport au reste de la surface (5).

8. Implant d'ostéosynthèse selon la revendication 1, 2, 3, 4, 5, 6 ou 7, caractérisé en ce qu'au moins l'une des faces partielles (4a, b) et la surface restante (6) présentent une surface d'enveloppement constante commune (21) dans la direction longitudinale de la tige.

9. Implant d'ostéosynthèse selon la revendication 1, 2, 3, 4, 5, 6, 7 ou 8, caractérisé en ce que les éléments de détail moulés (19) se superposent à la surface partielle (7) en retrait par rapport au reste de la surface (6), en donnant naissance à une cavité (20).

10. Implant d'ostéosynthèse selon l'une ou plusieurs des revendications qui précèdent, caractérisé en ce que la face partielle (4a) situé du côté à courbure convexe présente une longueur de 25-60 % de la longueur de la tige.

11. Implant d'ostéosynthèse selon l'une ou plusieurs des revendications qui précèdent, caractérisé en ce que la face partielle (4b) située du côté à courbure concave présente une longueur de 15-40 % de la longueur de la tige.

12. Implant d'ostéosynthèse selon l'une ou plusieurs des revendications qui précèdent, caractérisé en ce qu'il est coulé, de préférence d'une seule pièce, dans un matériau bio-inerte, en particulier un alliage de titane.

13. Implant d'ostéosynthèse selon l'une ou plusieurs des revendications qui précèdent, caractérisé en ce que les faces partielles structurées et les faces restantes sont agencées de façon à ce que l'implant puisse être soumis à un essai non destructif des matériaux.

14. Implant d'ostéosynthèse selon l'une ou plusieurs des revendications qui précèdent, caractérisé en ce que les éléments de détail moulés (8) présentent des hampes (14) de formes différentes et/ou des têtes (16) de formes différentes.
